# EUROPEAN PATENT APPLICATION

(11) **EP 4 152 339 A1**
(43) Date of publication of application: **22.03.2023**
(21) Application number: 21197827.5
(22) Date of filing: 20.09.2021
(51) Int. Cl.: G16H 30/40

(54) **METHOD FOR DETERMINING A SURGERY PLAN BY MEANS OF A REINFORCEMENT LEARNING METHOD**

(71) Applicant: Universität Zürich, 8006 Zürich (CH)
(72) Inventor: FÜRNSTAHL, Philipp, 8193 Eglisau (CH)
(74) Representative: Schulz Junghans Patentanwälte PartGmbB

(57) **Abstract**

The invention relates to a computer-implemented method for training an agent of a reinforcement learning method for determining a surgery plan, particularly a surgical access and manipulation of a tissue, the method comprising the steps of:
a) generating (200) a three-dimensional analytical representation (2) corresponding to a parametrized segmentation of a tissue portion, wherein the segmentation of the tissue portion subdivides the tissue portion in kinds of tissue of the tissue portion,
b) generating (300) from the analytical representation (2) a corresponding state space configured and designed for the reinforcement learning method to train the agent (1), wherein the state space comprises a plurality of states (s), wherein each state (s) comprises an information on its associated location in the tissue portion and an information indicative of at least the kind of tissue in the tissue portion it represents,
c) performing a training (400) of the agent (1) in the analytical representation (2) and the state space by means of the reinforced learning method to obtain a trained agent, wherein the training is performed by repeating step b) several times, wherein for at least some repetitions of step b) the analytical representation (2) is varied by adjusting the parametrized segmentation by way of adjusting at least one parameter of the parametrized segmentation to obtain an analytical representation (2) of a varied tissue portion.

## Description

The invention relates to a method for determining a surgery plan, particularly a surgical access and manipulation in a tissue portion by means of a trained agent of a reinforcement learning method, as well as to a method for training of the agent. Further, the method relates to a corresponding computer-implemented method as well as to a corresponding computer-program and computer program product. Reinforcement learning methods have been applied for incision and robotics in laparoscopy [1] (Baek, D., et al (2018) "Path Planning for Automation of Surgery Robot based on Probabilistic Roadmap and Reinforcement Learning."; pages 342-347. DOI: 10.1109/URAI.2018.8441801). The authors of [1] teach the planning of a path for robotic tools such that a collision of the robotic tools and surrounding tissue (in which no surgical intervention is planned) is avoided. The method disclosed in [1] comprises real-time resection/suturing and the use of probabilistic roadmap for path planning in binary two-dimensional images.

One problem in applying reinforcement learning methods in the surgical domain is to obtain sufficient large data sets for training an agent and its policy. For application of the trained agent, the data sets have to be sufficiently similar in order for the agent to perform adequately. This prerequisite however is difficult to achieve, as it requires the provision of training data that is similar enough to recorded image data. Recorded image data in turn is often times too noisy and does include information necessary for awarding the rewards to the agent during training. Therefore, as of yet, path planning in surgical interventions relying on an agent trained by a reinforcement learning method has not been achieved reliably.

In order to address surgical panning, alternative solutions have been proposed. For example, US10255723 B2 discloses a method for imaging and planning a surgical access in tissue by means of least costs approaches for determining the best path in the tissue, wherein the least cost approaches are unrelated to reinforcement learning methods.

The lack of a sufficiently large pool of training data for training the agent that at the same time allows the trained agent to successfully be applied to image data recorded from a patient is still an unsolved problem.

An object of the present invention is to provide a computer-implemented method for training and application of a reinforcement learning method for surgical planning. The object is achieved by the device having the features of claim 1.

Advantageous embodiments are described in the dependent claims.

According to a first aspect of the invention, a computer-implemented method for training an agent of a reinforcement learning method for determining a surgery plan of a tissue, the method comprising the steps of:
a) generating a three-dimensional analytical representation corresponding to a parametrized segmentation of data representative of a tissue portion, wherein the segmentation of the tissue portion subdivides the tissue portion in kinds of tissue of the tissue portion,
b) generating from the analytical representation a corresponding state space configured and designed for the reinforcement learning method to train the agent, wherein the state space comprises a plurality of states, wherein each state comprises an information on its associated location in the tissue portion and an information indicative of at least the kind of tissue in the tissue portion it represents,
c) performing a training of the agent in the analytical representation environment and state space by means of the reinforced learning method to obtain a trained agent, wherein the training is performed by repeating step b) several times, wherein at least for some repetitions, particularly for each repetition of step b) the analytical representation is varied by adjusting the parametrized segmentation by way of adjusting at least one parameter of the parametrized segmentation to obtain an analytical representation of an imaginary varied tissue portion, such that for each repetition a varied state space is obtained in step b).

The method according to the invention therefore provides a parametrized environment for the agent. As the analytical representation is parameterized it is possible to generate a very large pool of training data in form of analytical representations having varied the at least one parameter (typically the analytical representation comprises many parameters that are varied). This allows for the generation of well-trained agents by means of the reinforcement learning method. The variation of the at least one parameter (typically there are many parameters that are varied) particularly allows for simulating anatomical variations across individuals as well as pathologies.

By varying of the parameters particularly an anatomical appearance of the tissue portion is varied, such that the agent is trained on a plurality of different anatomies that may be encountered once the agent is trained. Variation of the at least one parameter may affect the size, density, shape and/or distribution of kinds of tissue of the tissue portion.

In terms of reinforcement learning, the analytical representation particularly corresponds to the environment in which the agent is to determine the surgery plan. The term "surgery plan" particularly refers to a surgical access and manipulation of tissue, e.g. by medical tools, such as scalpel, drill and/or saw.

The surgery plan typically comprises a starting point, e.g. on the skin tissue surface, and an end point to which a person skilled in the art wants to have an access. The starting point and the end point are particularly connected by a path, wherein the training of the agent aims to determine said path, particularly according to the best practice guidelines of surgery.

The term "agent" is used herein as used in the art relating to reinforcement learning methods. The agent may perform actions in the state space based on a policy that has been acquired or is formed during training. The policy allows the trained agent to determine a solution, in this case particularly a path connecting starting and end point, that is deemed optimal by the trained agent.

During training, the agent learns by means of a reward function and particularly by means of deep learning method, how to determine an optimal path for the surgery plan.

The quality of a trained agent depends on many parameters, such as the number of training data sets available, the quality of data sets for training and execution, similarity of training data and the data the agent is applied to and many more.

One advantage of the reinforcement learning method is that compared to other machine learning methods, such as artificial neural networks, it does not require the generation of reference solutions for the training datasets which is a difficult, timeconsuming and sometimes impossible task.

In case the training is to be based on image data acquired from a tissue sample, or a person, the image data may vary in terms of quality, noise and other hard to control parameters.

The invention solves this problem by generating an analytical representation that is a parametrized and segmented representation of the tissue portion of interest. By varying the at least one, typically many parameters of the parametrized analytical representation, it become possible to obtain as many data sets representative of imaginary tissue portions for training as necessary with a straight forward and computationally unexpensive approach.

Particularly, the variations of the at least one parameter, particularly the plurality of parameters, is configured such that the variations mimic the anatomical variation within the population and the one or more parameters are obtained or derived from literature or previous data.

According to another embodiment of the invention, for some repetitions the analytical representation is not varied but only an end position of the path determined by the agent is adjusted to a different end position with respect to a previous trial. Segmentation of the analytical representation allows for the generation of a defined state space including the kind of tissue that may be of relevance to the agent's decision making. Advantageously, the approach of a parametrized and segmented analytical representation allows also for using image data recorded from tissue portions by segmenting and parametrizing the image data, such that an analytical representation is obtained that has the same characteristics as the analytical representation of claim 1.

Thus, the analytical representation may be obtained from recording of image or sensor data from a tissue portion or by a purely artificial/synthetic generating approach. Independently of the way of obtaining the analytical representation, it is then possible to vary the analytical representation by varying the at least one parameter in order to generate more analytical representations.

The state space is associated to the analytical representation in such a way that for each sequence of states that is selected by the agent a corresponding path in the analytical representation may be determined.

The kinds of tissue may be selected from a group of tissues, wherein not necessary all kinds of tissue need to be represented by the analytical representation. Particularly, a state of the state space may comprise only one kind of tissue. According to another embodiment of the invention, the analytical representation comprises or is formed by a plurality of geometric primitives, each primitive being parametrized by at least one parameter and associated to one kind of tissue of the tissue portion, particularly wherein in step c) of claim 1 the parameters of at least some of the geometric primitives are varied in order to obtain a varied analytical representation of a varied tissue portion.

The use of geometric primitives is well-known in CAD applications and allow for parametrizing or vectorization of even complex image data consistently. Particularly, in case the analytical representation is obtained from recorded sensor or image data, first the image data is segmented and then parametrized.

The geometric primitives are particularly three-dimensional geometric primitives. According to another embodiment of the invention, a CAD method, i.e. a computeraided design method is used for parametrizing the tissue portion, particularly wherein the CAD method is used for generating the geometric primitives comprised by the analytical representation and/or for varying the analytical representation, particularly by varying the parameters of the geometric primitives.

This embodiment allows for near-automatic or automatic generation of the parametrized analytical representation.

According to another embodiment of the invention, the kind of tissue is selected from a group comprising one or more of: bone tissue, muscle tissue, fascia tissue, nerve tissue, blood vessel, skin tissue.

These kinds of tissue may be of particular relevance for determining a surgery plan. For example, ideally nerve tissue should not be cut, while muscle and fascia tissue are kinds of tissue that can be cut during surgery.

Bone tissue on the other hand might require different surgical tools in order to penetrate.

According to another embodiment of the invention, for training, the agent selects from a plurality of actions in an action space representative for positioning a medical tool in the tissue, and/or representative for an action performed by the tool, such as cutting the tissue, repositioning the tissue or tool, drilling through the tissue, and/or fixing.

The state space, the action space, the analytical representation and the reward function form the so called "environment" for the agent.

According to another embodiment of the invention, for training, comprised by step b), the method comprises the step b1) of initiating the agent at a starting position in the state space that corresponds to a target location in the tissue portion to which the surgery plan is aimed.

This embodiment allows a dramatically improved training of the agent in terms of time and number of trials, as most importantly the target location is always reached by the agent, as it forms the starting point of the agent.

According to another embodiment of the invention, the training comprises manipulation, reorganization of tissue represented by the analytical representation. Particularly, the agent may select actions (of an action space) that allow for relocating tissue, such as bone tissue.

These actions may comprise, cutting, drilling, removing tissue and/or relocating tissue.

Particularly, the agent may be trained solely for manipulation of tissue and not for determining a path to the target location as will be disclosed in the following. According to another embodiment of the invention, the reward is calculated from the action selected by the agent and the resulting state transition, and the reward function. According to another embodiment of the invention, in a step b2) subsequent to step b1) and comprised in step b) from the starting position, particularly an interconnected sequence of neighbouring states in the state space is determined by the agent, particularly by means of the selected actions in the action space, wherein the sequence of states in the state space corresponds to a geometrical path consisting of interconnected path segments in the analytical representation of the tissue such that the determined sequence of states represents a path in the analytical representation.

This embodiment details the connection of the state space with the analytical representation and a path that is determined by the agent in the analytical representation.

The sate space can be multidimensional and thus the term "neighbouring" relates to adjacent states in the said multidimensional state space.

According to another embodiment of the invention, for each state of the sequence of states in the state space that is selected by the agent, a reward value is awarded to the agent by a reward function of the reinforcement learning method, wherein the reward function depends on the selected state, at least on the kind of tissue that is comprised by the corresponding path segment, a distance of the path to at least one selected, e.g. critical kind of tissue, such as nerve tissue and/or blood vessel tissue, a length of the path, a curvature of the path in the analytical representation, and/or the path arriving at an end position of the surgery plan, wherein the end position corresponds to a state in the state space being associated to skin tissue. Particularly, the longer the path, the lower the reward. The same applies to the curvature of the path: The higher the curvature, the lower the reward.

This way, comparably straight and short paths are favoured over long and curvy path.

Similarly, a path that does not end on the skin tissue (end position) is rewarded so low that a trained agent will not suggest a path that does not end on skin tissue. Further, the higher a distance from the path to critical tissue such as nerves and/or blood vessels the higher the reward. Cutting through a nerve or blood vessel is associated with a very low reward, such that paths extending too close or through critical kinds of tissue are avoided by the agent.

Particularly, the parametrized analytical representation allows for facile determination of a distance, as compared to other representations where distance metrics are hard to establish.

This embodiment allows for the provision of rewards to the agent based on best practice guideline of surgery (e.g. short and straight cuts that do not cut important nerves or essential blood vessels).

According to another embodiment of the invention, the path segments and the path have a geometrical extent that corresponds to a geometrical footprint of a medical tool applied to the tissue, such as a cut shape of a scalpel, a drill-hole shape of a medical drill or a cut-shape of a saw, particularly wherein the path segments and the path in the analytical representation have a planar, a ribbon-shaped or a tube-shaped, particularly a cylindrical extent in the analytical representation of the tissue portion, particularly, wherein the extent of the planar, ribbon- or tube-shaped path has a width or a diameter that accounts for an extent and/or shape of an medical tool, particularly wherein the width of the path extends perpendicular to the path. This embodiment takes care of the finite width and shape of the surgical tool, when e.g. performing an incision or drilling, such that the determined path reflects the medical tool used.

According to another embodiment of the invention, the reward is calculated from the action selected by the agent and the resulting state transition, the path in the analytical representation and the reward function.

According to another embodiment of the invention, the reinforcement learning method is a deep reinforcement learning method.

The deep reinforcement learning allows to obtain a particularly robust and well-trained agent.

According to another embodiment of the invention, the agent is trained using a deep neural network.

This embodiment particularly refers to the agent's policy being optimized by applying an artificial neural network.

According to another embodiment of the invention, the analytical representation with the features of step a) is generated from recorded sensor or image data of the tissue portion.

This embodiment allows the use of recorded image data from tissue portions for training or at least for providing an initial analytical representation that subsequently is varied by varying the at least one parameter of the analytical representation. According to another embodiment of the invention, for generating the analytical representation from the sensor or image data, a computer-implemented segmentation and/or a computer-implemented classification method assigns the signals, that is for example comprised in voxels of the sensor- or image data, recorded in the sensor or image data to the kinds of tissue such that a segmented representation of the tissue portion is obtained from the sensor or image data, wherein the segmented representation is subsequently parametrized, particularly in form of the geometric primitives.

This embodiment allows for creating analytical representations from measurements on patients or tissue portions for training of the agent. This approach ensures consistent quality and characteristics of the analytical representation independently of its origin, e.g. derived from sensor- or image data or formed by varying the at least one parameter of an existing analytical representation.

According to another embodiment of the invention, the image data are recorded by a medical imaging device method, particularly wherein the image data are composed from CT-data, MRI-data, X-ray-data, and/or from ultrasound-data.

This embodiment allows for the application to use common imaging modalities used in medical imaging.

According to another embodiment of the invention, the analytical representation of step a) is generated by the steps of:
- In a coordinate system generating bone and cartilage tissues voxels representing bone tissue extending along a first direction, particularly wherein the bone tissue has a cylindrical shape of radius r1, wherein the radius is greater than one voxel,
- Generating skin tissue voxels representing skin tissue circumferentially extending around the bone tissue along the first direction forming a space between the bone tissue voxels and the skin tissue voxels, particularly wherein the skin tissue has a cylindrical shape of radius r2, wherein the radius is greater than the radius of the bone tissue,
- Generating in the space between skin and bone tissue voxels, muscle tissue voxels representing muscle tissue, wherein the muscle tissue comprises a plurality of spindle-shaped muscles that extend along the first direction and wherein the muscles have a width, particularly a varying width along a plane perpendicular to the first direction,
- Generating fascia tissue voxels representing fascia tissue that are in direct contact with the muscle tissue voxels, particularly wherein the fascia tissue has a thickness of one voxel only, particularly wherein the fascia tissue surrounds the muscle tissue at least partially,
- Generating nerve tissue voxels representing nerve tissue, particularly wherein the nerve tissue comprises a plurality of branching nerve bundles that extend through the muscle and the fascia tissue along the first direction,
- Parametrizing the voxels of the kinds of tissue to obtain the analytical representation.

This embodiment teaches one possibility of creating an artificial analytical representation that is not directly based on sensor or image data recorded form a tissue portion, but that is in essence simulated.

The bone tissue can be generated using any kind of anatomical bone shapes, such as hip bone shape, spinal chord shape etc. The tissue portion does not necessarily only comprise a cylindrical bone tissue section.

For generating the different kinds of tissue for the analytical representation, a computer program may be used that is configured to execute these steps. For this purpose, the computer program may be initialized with a set of parameters characterizing the imaginary tissue portion to be generated in terms of fractions, shapes and distribution the kinds of tissue.

This embodiment allows the creation of synthetic analytical representations suitable for training the agent.

The agent may be trained on synthetic analytical representations as well as on analytical representation generated from sensor or image data as described in previous embodiments as well as form analytical representations that are generated by varying the at least one parameter of either one of the analytical representations. According to another embodiment of the invention, the target location for the surgery plan is provided to the method, e.g. by a user of the method or the type of surgery to be executed, and wherein the trained agent determines the sequence in the state space - that after training corresponds to an optimal state sequence and thus an optimal path in the analytical representation - wherein the analytical representation is generated from recorded sensor or image data.

This embodiment allows for the application of the trained agent to recorded sensor or image data for determining a surgery plan for the planned intervention.

According to another embodiment of the invention, the sequence of states is determined and the corresponding path in the analytical representation from the starting position that corresponds to the target location, i.e. to an end position of the surgery plan.

This embodiment allows for a more efficient application of the trained agent to the analytical representation, particularly wherein during training the agent has been initialized also at the target position of the surgical plan, such that a high degree of consistency between training and application of the agent is achieved.

According to another embodiment of the invention, the determined corresponding path of the trained agent is transferred from the analytical representation back to the sensor or image data, particularly wherein the determined corresponding path is visualized in the sensor or image data and/or wherein the determined corresponding path is transferred to a surgical navigation system.

This embodiment provides a fully integrated application of the trained agent to a surgical and/or an orthopedic intervention that may be executed by a skilled medical person. Further, this embodiment also covers the application of the trained agent and its capabilities in surgical navigation systems, that are configured to aid a surgeon during surgery e.g. by displaying a position and/or orientation of the medical tool in use relative to the determined path.

According to a second aspect of the invention, a computer-implemented method for determining a surgery plan, particularly a surgical access and manipulation in a tissue portion by means of a trained agent of a reinforcement learning method, particularly of a deep reinforcement learning method, is provided, wherein the computer-implemented method comprising the steps of:
- Acquiring sensor or image data from the tissue portion,
- From the sensor or image data generating a three-dimensional analytical representation corresponding to a parametrized segmentation of the sensor or image data of the tissue portion, wherein the segmentation subdivides the sensor or image data of the tissue portion in kinds of tissue of the tissue portion,
- Generating from the analytical representation a state space configured and designed for the reinforcement learning method with a plurality of states, wherein each state comprises an information on its associated location in the tissue portion and an information indicative of at least the kind of tissue in the tissue portion it represents,
- Initiating the agent at a starting position in the state space,
- From the starting position determining by the trained agent a sequence of states in the state space, particularly, wherein the sequence of states corresponds to interconnected path segments forming a path and/or wherein the sequence of states corresponds to actions regarding a tissue manipulation of the tissue represented in the analytical representation,
- Transferring the determined surgery plan, e.g. the determined path or the sequence of manipulations by the trained agent to the recorded sensor or image data,
- Particularly displaying the determined surgery plan in the image data to a person performing the surgery
- More particularly, performing surgery based on the surgery plan.

The method according to the second aspect of the invention, allows for determining a surgery plan based on an agent trained on analytical representations. The application of a such trained agent allows for determining surgery plans that are in accordance with surgical guidelines, while the path is determined essentially autonomous by the trained agent, without interference of a person.

Terms and definitions from the first aspect of the invention, are applicable to the second aspect of the invention. Particularly embodiments and features relating to the first aspect of the invention are applicable to the second invention as well, particularly regarding the training procedure of the agent as well as the generation of the analytical representation.

Particularly, the agent may select actions (of an action space) that allow for relocating tissue, such as bone tissue.

These actions may comprise, cutting, drilling, removing tissue and/or relocating tissue.

According to another embodiment of the invention, the starting position in the state space corresponds to a target location in the tissue portion to which the surgery plan is aimed.

According to another embodiment of the invention, the determined path arrives at an end position of the surgery plan, wherein the end position corresponds to a state in the state space being associated to skin tissue.

According to another embodiment of the invention, the agent is trained by means of the computer-implemented method according to the first aspect of the invention.

According to another embodiment of the invention, a template analytical representation from the tissue portion is provided, wherein the sensor or image data are segmented by a segmentation method assigning the kinds of tissue in the tissue portion to the segmentation, wherein the template analytical representation is fitted to the segmented sensor or image data by adjusting the at least one parameter of the template analytical representation such that the analytical representation of the tissue portion is generated.

This embodiment allows to generate an analytical representation corresponding to the recorded sensor or image data by adjusting an existing, namely the template analytical representation, particularly by adjusting its at least one parameter. According to a third aspect of a computer program and/or a computer program product comprising or having stored thereon the computer program, is provided, wherein the computer program is configured to execute the computer-executable steps of the method according to the first and/or second aspect of the invention. According to a fourth aspect of the invention the method is configured to be executed on a computer or a computer network, having at least one processor as well as a data memory system that is in communication with the at least one processor, wherein the analytical representation, the sensor or the image data may be stored in the memory system, and wherein the processor generates the state space and performs the training of the agent and/or the application of the trained agent to the analytical representation.

It is noted that training and applying an agent from a reinforcement learning method, particularly a deep reinforcement learning method requires a high-performance computer, in order to cycle through the plurality of cycles required for training. According to an embodiment of this aspect, the computer or computer network is connected to at least one display configured to display the analytical representation, the recorded sensor or image data with at least one path segment or the complete path arranged correspondingly on the display, particularly as a graphical overlay on the analytical representation, or the sensor- or image data.

According to a fifth aspect of the invention, the determined path is applied to a surgical intervention on a patient, wherein the surgical intervention is executed along the determined path of the agent.

Particularly, exemplary embodiments are described below in conjunction with the Figures. The Figures are appended to the claims and are accompanied by text explaining individual features of the shown embodiments and aspects of the present invention. Each individual feature shown in the Figures and/or mentioned in said text of the Figures may be incorporated (also in an isolated fashion) into a claim relating to the device according to the present invention.

The method according to the invention allows to find an optimal path for a surgical access, given a 3D-voxel-space spanned by a patient's CT. The method allows to train an agent via a reinforcement learning method whose policy is to optimally navigate from skin tissue, i.e. the patient surface, to a predetermined target location by following best practice methods used by surgeons.

While the method may be used on incisions for open orthopedic surgery other applications, where surgical planning is of importance are well within the scope of the present invention.

The method uses a synthetically generated analytical representations of a tissue portion, on which an agent of the reinforcement learning method is trained.

The problem on generating or obtaining a sufficiently large number of training data and later the problem of how to unify sensor- or image data recorded from a tissue portion is addressed by the method according to the invention.

The synthetically generated analytical representations of the tissue portion allow for the cost-efficient training of the agent and enables the introduction of a simple anatomical model of the tissue portion that can be extended further on to capture varying aspects of the tissue portion. Particularly, the analytical representation comprises some of the surgically most relevant structures in the human body.

Amongst these, there is skin tissue, bone tissue, nervous tissue, muscle tissue, and fascias. The inclusion of these structures allows the most critical decision making when searching for an optimal approach for surgery. The bones and muscles make up the musculoskeletal system and thus often times represent the target areas for surgical plans. The fascias fill the intermuscular space and consist of connective tissue which can be cut and sewn relatively easily: the fascial tissue forms the main passage route, wherein damage to nervous tissue may be irreversible which is why one aims to spare nerves during the incision.

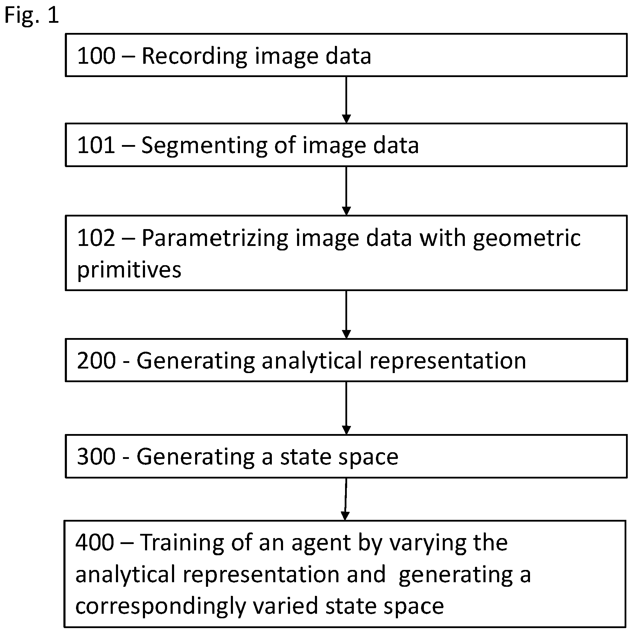

In Fig. 1 an exemplary training procedure is shown in form of a flow-chart. In a first step, image- or sensor data from a tissue portion is recorded 100. The image data may be obtained from a CT-measurement. This image data is then segmented by a suitable segmenting method 101, that segments the image data according to the underlying kind of tissue. After the image data is segmented, the segmented image data may comprise tissue portions segmented in muscle, nerve, fascia, bone, blood vessel and skin tissue. Other kinds of tissue may be recognized and segmented accordingly by the segmentation method.

Subsequently, the segmented image data is parametrized 102 for example by means of vectorizing in geometric primitives that allow a precise yet efficient parametrization of the segmented image data. The such segmented and parametrized image data forms 200 the analytical representation of the tissue portion. Parametrizing 102 the segmented image data may be done with a CAD program that finds the geometric primitives for describing the segmented image data correctly. By changing the parameters of the geometric primitives, it is possible to adjust and change the analytical representation.

Alternatively, the analytical representation may be obtained "form the scratch" by generating the segmented image data completely without sensor- or image data, with an appropriate program for designing artificial tissue portions. This approach has the advantage that the tissue portion is assigned with the correct kinds of tissue at all times.

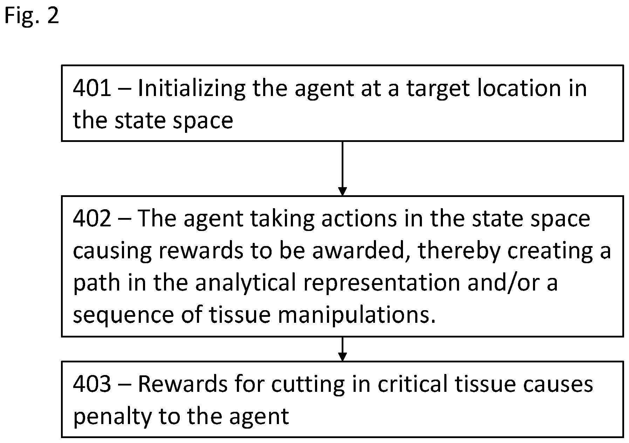

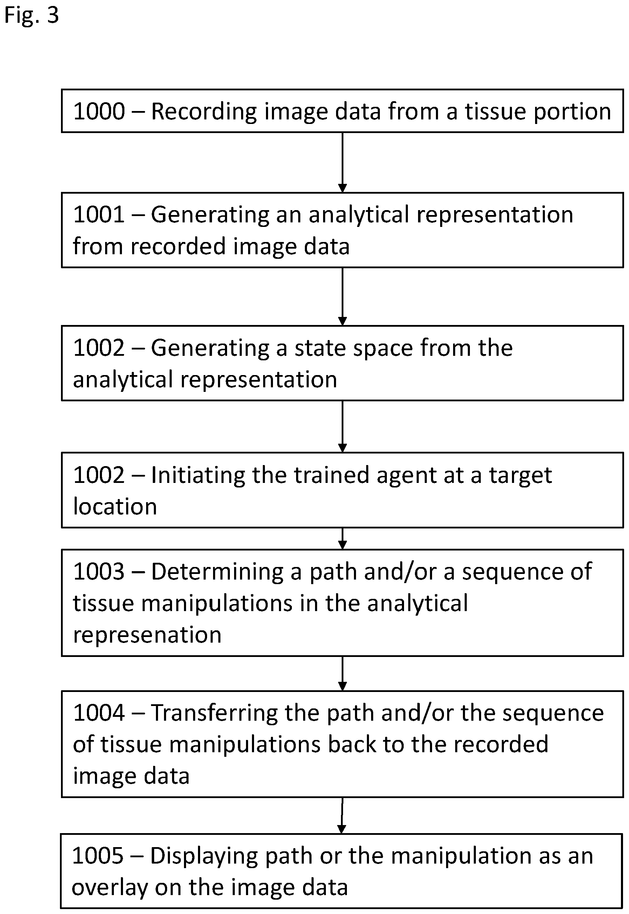

Independently of how the analytical representation is generated initially, a first trial of the training may be executed 300 in a state space with a plurality of states generated from the analytical representation. The agent takes actions 402 (cf. Fig. 2) in the state space wherein a reward is attributed to the action the agent chose and the state the agent ends in after the action.

For this, the agent is initialized at the target location 401 (cf. Fig. 2) of the surgery plan, and essentially finds its way backward to the skin tissue.

Rewards for cutting 403 through critical tissue or not ending the path on skin tissue are awarded in form of comparably high penalties, e.g. in form of a negative reward awarded to the agent.

While the agent tries to find an optimal sequence of states in the state space, said sequence is associated to a path in the analytical representation, wherein said path comprises path segments that have an extend and a shape in the analytical representation. Particularly, as the analytical representation comprises a plurality of is geometric primitives determining for example a distance between the primitives, and thus any kind of tissue and the path in the analytical representation is computationally well-defined and unambiguous. The extent of the path may resemble the form of a cut made by a surgical tool, e.g. a scalpel. In this case the shapes of the path segments are most likely ribbon-like, wherein when a drill is used, the shapes are cylindrical or tube-shaped.

The agent's goal is to reach the skin tissue, under the premises of maximizing the cumulative rewards along the path in the analytical representation.

Training of the agent is facilitated by means of a deep learning method such as an artificial neural network that is configured to determine the agent's policy derived from the rewards earned from the varied analytical representation 400 and state spaces in which the agent has been trained.

One example that is successfully addressable by the method according to the invention, is to define the scalpel cutting path through different tissues (skin, fat, muscle). The surgeon is particularly interested to identify the path between skin incision and a certain region (target location) defined on a particular anatomy like a bone.
- S represents the finite set of possible states which are 3-tuples (analytical representation, agent position and set of visited positions - i.e. the ongoing cut). In particular, each state s can be represented in a single image by encoding the agent and the visited positions directly into the image. In the initial state s_0, the agent is positioned at the desired region, i.e. target location on the bone for which the cutting path should be calculated. The state space has several goal states *s_g1, s_g2,* ... which are distributed on the "skin" tissue layer, as the objective of the agent is to find the least invasive pass from s_0 to any s_g.
- A represents a finite set of actions a that allow the agent to interact with the environment. Each action a consists to a *"scalpel cut* defined by a discretized direction (e.g., 24-neighborhood) and a discrete step-size (e.g., 1 mm)
- R (s_i, a_i) is a scalar reward function which attributes a reward when moving from s->s' using action a. The reward gives small penalties (-1) to favor straight and simple cuts when cutting through desired tissues. High penalties are given when cutting through critical structures like nerves/vessels (-50). A high final reward is given when the agent achieves to reach one of the final states.

Another example is the surgical planning of corrective bone osteotomies which are performed with a surgical saw. The analytical representations consist of an ellipsoid EP (representing the pathological bone anatomy), a sphere G (representing the reconstruction template derived from the contralateral bone), Gaussian processes (representing bone regions of special importance such as necrotic bone, or vessel insertation sites EN_i) and analytical plane equations P1 and P2 (representing the osteotomy cuts). The objective of the agent is to learn how to reconstruct the spherical shape of the bone by performing two osteotomies followed by bone realignment.
- A state *s_i* is modelled by continuous parameters encoding the steps of the surgery: 3D plane parameters for the first osteotomy, 3D-plane parameters for the second osteotomy, and a rigid 3D transformation for the bone realignment.
- An action is taken by the agent to change and optimize the states towards the optimal surgical outcome. Action *a*_*i* is of equal length than s_i and consists of deltavalues corresponding to a parameter change.
- The reward function consists of 4 parts: The first part quantifies the sphericity of the resulting femoral head. The second part controls how much of the patholical bone is resected and how much of the healthy bone remains. The third part penalizes cuts which go through artery insertion sites. The last part controls the quality of the fragment realignment by minimizing the intra-articular step-off. The range of reward values in this example is from -50 to 2.

Concrete numbers about training hyperparameters, size of the training data and number of primitives, etc. are very dependent on the use case. In the above examples, 1-2 million of iterations are required until the agents learning curve becomes stable. The data set consists of several thousands of anatomical instances. Training can be performed within days on state-of-the-art PCs or accelerated by cloud computing platforms.

### Application of a trained agent to image data:

Another aspect of the invention relates to the application of the trained agent to recorded image data 1000 of a tissue portion on which a surgical intervention is planned.

As the agent has been trained on segmented and parametrized analytical representations, it is necessary to transfer the recorded image in such an analytical representation 1001.

This way, the agent will operate most robust and reliable. For this purpose, a segmentation and parametrizing method may be applied. Segmentation has to be such that kinds of tissue are assigned to the image data. Further, parametrization may be obtained by vectorizing the image data, e.g. by means of geometric primitives.

The trained agent is the initiated 1003 in the state space associated 1002 to the analytical representation, at a target location of the planned intervention. The target location has to be provided by a user of the method.

The agent then finds 1003 the path based on his trained policy an optimal sequence of states in the state space which corresponds to an optimal path in the analytical representation. The path connects skin tissue with the target location, while taking into account the anatomy reflected in the analytical representation, kinds of tissue, and path properties such as curvature, distance to critical tissue, such as nerves and or blood vessels.

Once the path is determined by the trained agent, the path is transferred back 1004 to the image data. There the path may be displayed 1005 to a user or it may be loaded into a computer program for surgical navigation.

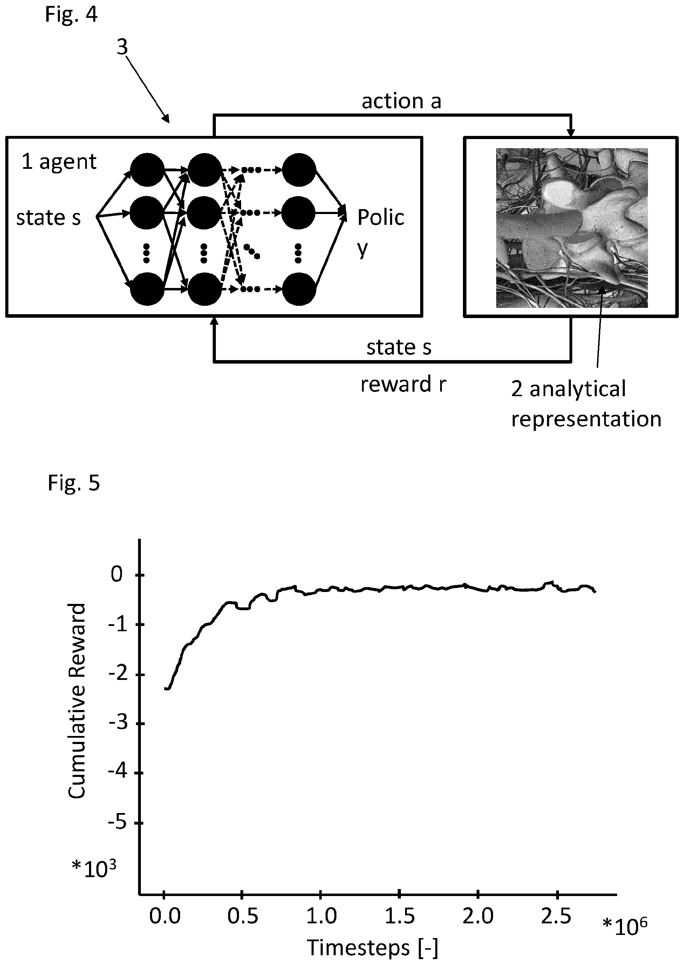

Fig. 4 shows a schematic diagram of how training of the agent is facilitated. The agent 1 is trained in a state space that is generated from the segmented and parametrized analytical representation 2. The analytical representation 2 is representative for a tissue portion with different kinds of tissue. As can be seen, the analytical representation 2 in Fig. 4 comprises bones, cartilage tissue, nerves and blood vessels. The agent 1 may transission from one state to another state in the state space by means of an action a chosen from an action space. The new state s and the action is rewarded with a reward rand provided to the agent. This way a policy of the agent is trained, e.g. by means of a neural network 3 which is schematically shown in Fig. 4. The agent's goal is to maximize the cumulative reward. During training the agent's policy is optimized, such that the trained agent repeatedly scores a high cumulative reward.

In Fig. 5 a training progress of an agent is shown. Here the cumulative reward value is plotted versus time steps, i.e. training sessions. The training session may comprise a plurality of varied analytical representations. As can be seen the cumulative reward stabilizes between 0.5 and 1 million time steps.

## Claims

1. A method, particularly a computer-implemented method for training an agent of a reinforcement learning method for determining a surgery plan, particularly a surgical access and manipulation of a tissue, the method comprising the steps of:
a) generating (200) a three-dimensional analytical representation (2) corresponding to a parametrized segmentation of a tissue portion, wherein the segmentation of the tissue portion subdivides the tissue portion in kinds of tissue of the tissue portion,
b) generating (300) from the analytical representation (2) a corresponding state space configured and designed for the reinforcement learning method to train the agent (1), wherein the state space comprises a plurality of states (s), wherein each state (s) comprises an information on its associated location in the tissue portion and an information indicative of at least the kind of tissue in the tissue portion it represents,
c) performing a training (400) of the agent (1) in the analytical representation (2) and the state space by means of the reinforced learning method to obtain a trained agent, wherein the training is performed by repeating step b) several times, wherein for at least some repetitions of step b) the analytical representation (2) is varied by adjusting the parametrized segmentation by way of adjusting at least one parameter of the parametrized segmentation to obtain an analytical representation (2) of a varied tissue portion.

2. The method according to claim 1, wherein the analytical representation (2) comprises a plurality of geometric primitives, each primitive being parametrized by at least one parameter and associated to one kind of tissue of the tissue portion, particularly wherein in step c) the parameters of at least some of the geometric primitives are varied so as to obtain a varied analytical representation (2) of a varied tissue portion.

3. The method according to any of the preceding claims, wherein for training (400), comprised by step b), the method comprises a step b1) of initiating the agent (1) at a starting position in the state space that corresponds to a target location in the tissue portion to which the surgery plan is aimed.

4. The method according to claim 3, wherein in a step b2) subsequent to step b1) and comprised in step b) from the starting position, a sequence of states in the state space is determined by the agent (1), particularly by means of the selected actions (a) of an action space in the state space, wherein the sequence of states (s) in the state space corresponds to a geometrical path consisting of path segments in the analytical representation of the tissue such that the determined sequence of states represents a path in the analytical representation, and/or particularly wherein the sequence of states corresponds to a sequence of tissue manipulations.

5. The method according to claim 4, wherein for each state of the sequence of states in the state space that is selected by the agent, a reward value is awarded to the agent by a reward function of the reinforcement learning method, wherein the reward function depends on the selected state, at least on the kind of tissue that is comprised by the corresponding path segment, a distance of the path to at least one selected kind of tissue, such as nerve tissue and/or blood vessel tissue, a length of the path, a curvature of the path in the analytical representation, and/or the path arriving at an end position of the surgery plan.

6. The method according to claim 4 or 5, wherein the path segments and the path have a geometrical extent that corresponds to a geometrical footprint of a medical tool applied to the tissue, such as a cut shape of a scalpel, a drill-hole shape of a medical drill or a cut-shape of a saw, particularly wherein the path segments and the path in the analytical representation have a planar, a ribbon-shaped or a tube-shaped, particularly a cylindrical extent in the analytical representation (2) of the tissue portion, particularly, wherein the extent of the planar, ribbon- or tube-shaped path has a width or a diameter that accounts for an extent and/or shape of an medical tool, particularly wherein the width of the path extends perpendicular to the path.

7. The method according to any of the preceding claims, wherein the analytical representation (2) is generated (200) from recorded (100) sensor or image data of the tissue portion.

8. The method according to any of the preceding claims, wherein the target location for the surgery plan is provided to the method, and wherein the trained agent determines the sequence in the state space, wherein the analytical representation is generated from recorded sensor or image data.

9. The method according to claim 8, wherein the sequence of states is determined as well as the corresponding path in the analytical representation (2) from the starting position that corresponds to the target location and/or as well as a manipulation of tissue represented by the analytical representation (2).

10. The method according to claim 9, wherein the determined corresponding path and/or the issue manipulation is transferred to the sensor or image data, particularly wherein the determined corresponding path and/or the manipulation of tissue is visualized in the sensor or image data and/or wherein the determined corresponding path and/or the manipulation of tissue is transferred to a surgical navigation system.

11. A method, particularly a computer-implemented method for determining a surgery plan, particularly a surgical access and/or a manipulation in a tissue portion by means of a trained agent of a reinforcement learning method, particularly of a deep reinforcement learning method, the computer-implemented method comprising the steps of:
- Acquiring (1000) sensor or image data from the tissue portion,
- From the sensor or image data generating (1001) a three-dimensional analytical representation corresponding to a parametrized segmentation of the sensor or image data of the tissue portion, wherein the segmentation subdivides the sensor or image data of the tissue portion in kinds of tissue of the tissue portion,
- Generating (1002) from the analytical representation a state space configured and designed for the reinforcement learning method with a plurality of states, wherein each state comprises an information on its associated location in the tissue portion and an information indicative of at least the kind of tissue in the tissue portion it represents,
- Initiating (1002) the agent at a starting position in the state space,
- From the starting position determining (1003) by the trained agent a sequence of states in the state space, particularly wherein the states correspond to interconnected path segments forming a path and/or a manipulation of the tissue represented in the analytical representation,
- Transferring (1004) the determined surgery plan by the trained agent to the recorded sensor or image data.

12. The method according to claim 11, wherein the starting position in the state space corresponds to a target location in the tissue portion to which the surgery plan is aimed.

13. The method according to any of the claims 11 or 12, wherein in case the surgical plan comprises a path, the determined path arrives at an end position of the surgery plan, wherein the end position corresponds to a final state in the state space.

14. The method according to any of the claims 11 to 13, wherein the agent is trained by means of the computer-implemented method according to any of the claims 1 to 10.

15. The method according to any of the claims 11 to 14, wherein a template analytical representation from the tissue portion is provided, wherein the sensor or image data are segmented by a segmentation method assigning the kinds of tissue in the tissue portion to the segmentation, wherein the template analytical representation is fitted to the segmented sensor or image data by adjusting the at least one parameter of the template analytical representation such that the analytical representation of the tissue portion is generated.
